# FASCICULE DE BREVET EUROPEEN

(11) **EP 1 082 451 B1**
(45) Date de publication et mention de la délivrance du brevet: **28.06.2006**
(21) Numéro de dépôt: 99919158.8
(22) Date de dépôt: 30.03.1999
(51) Int. Cl.: C12Q 1/00

(54) **PROCEDE POUR LA DETERMINATION D'ANTIBIOTIQUES A NOYAU BETA-LACTAME DANS UN LIQUIDE BIOLOGIQUE**
VERFAHREN ZUR BESTIMMUNG DES BETA-LACTAM NUKLEUS ENTHALTENDEN ANTIBIOTIKUMS
METHOD FOR DETERMINING ANTIBIOTICS WITH BETA-LACTAM CORE IN A BIOLOGICAL FLUID

(30) Priorité: 25.06.1998 BE 9800485
(43) Date de publication de la demande: 14.03.2001
(73) Titulaire: NEOGEN CORPORATION, Lansing, MI 48912 (US)
(72) Inventeur: DEGELAEN, Jacques, B-1470 Genappes (BE); GRANIER, Benoît, B-4120 Rotheux (BE); FRERE, Jean-Marie, B-4550 Nantrin (BE); JORIS, Bernard, B-4900 Spa (BE)
(74) Mandataire: Grünecker, Kinkeldey, Stockmair & Schwanhäusser Anwaltssozietät
(86) Numéro de dépôt international: PCT/EP1999/002166
(87) Numéro de publication internationale: WO 1999/067416

(56) Documents cités:
- US-A- 4 762 782

## Description

La présente invention se rapporte à de nouveaux procédés rapides et sensibles pour la détermination d'antibiotiques à noyau β-lactame dans un liquide biologique utilisant un récepteur sensible aux antibiotiques à noyau β-lactame de Bacillus licheniformis. Elle se rapporte également à des kits pour la mise en oeuvre de ces procédés.

A l'heure actuelle, les antibiotiques sont très largement utilisés non seulement comme agents thérapeutiques dans le traitement des maladies infectieuses provoquées par les bactéries, mais également comme agents de conservation des aliments et comme additifs dans la nourriture des animaux pour stimuler la croissance. La nécessité se fait donc de plus en plus sentir de pouvoir détecter la présence d'antibiotiques, même en très faibles concentrations, dans des liquides biologiques complexes, comme le lait, l'urine, le sang, le sérum, la salive, les extraits de viande, les liquides de fermentation, ou dans des milieux aqueux tamponnés.

Le cas de la production de lait en est un exemple. Il est bien connu en effet d'utiliser des antibiotiques pour traiter certaines maladies infectieuses du bétail producteur de lait.

Or, pour des raisons médicales évidentes, le lait destiné à la consommation humaine doit, en principe, être exempt de toute trace d'antibiotiques. Par ailleurs, des concentrations en pénicilline de 0,005 U.I./ml ou moins peuvent avoir des incidences néfastes lors de la fabrication de produits dérivés du lait comme le fromage, le yaourt, etc.

Plusieurs situations peuvent être envisagées. Dans un premier cas, par exemple pour détecter la présence d'antibiotiques à la ferme avant de transvaser dans un camion, la priorité sera donnée à un test extrêmement rapide (inférieur à 5 minutes) et simple. On peut également envisager d'utiliser un tel test rapide lorsque par exemple l'antibiotique qui a été utilisé pour le traitement est connu et que par ailleurs ce test permet la détection de l'antibiotique en question à la norme légale. Dans le deuxième cas, lorsque l'accent n'est pas mis sur la rapidité, l'importance est de détecter la majorité, si pas tous les antibiotiques aux normes légales.

La législation de certains pays impose en effet des normes de qualité bien précises. Par exemple, les autorités américaines exigent que les concentrations dans le lait des six antibiotiques suivants ne dépassent pas des valeurs bien précises : pénicilline, 5 ppb: ampicilline, 10 ppb; amoxicilline, 10 ppb; cloxacilline, 10 ppb; céphapirine. 20 ppb: ceftiofur. 50 ppb. L'Union Européenne impose, elle aussi, des normes de qualité: pénicilline 4 ppb; amoxicilline 4 ppb; ampicilline 4 ppb; cloxacilline 30 ppb; dicloxacilline 30 ppb; oxacilline 30 ppb; céphapirine 10 ppb; ceftiofur 100 ppb: cefquinone 20 ppb; nafcilline 30 ppb; céfazoline 50 ppb.

Il pourrait donc être intéressant de disposer d'un test qui permettrait de détecter la majorité des antibiotiques. Par ailleurs, au niveau de l'industrie laitière, on peut considérer qu'à défaut d'avoir un test présentant toutes les caractéristiques de rapidité; de sensibilité et de simplicité, un test qui permettrait d'allier au mieux ces trois paramètres, même s'ils ne sont pas totalement couverts, serait intéressant.

Différents types de tests ont déjà été proposés pour la détection d'antibiotiques à noyau β-lactame dans un liquide biologique. Ces tests utilisent généralement des méthodes de détection faisant appel à un agent de reconnaissance (récepteur ou anticorps) qui reconnaît spécifiquement l'antibiotique ou un analogue de cet antibiotique, et à un agent de marquage (radioélément, enzyme, agent fluorescent, etc.). En fonction des éléments choisis, on parlera de radio immuno essai (RIA), de radio récepteur essai (RRA), d'enzyme immuno essai (EIA) etc. Dans leur principe général, ces tests font appel à la combinaison minimale des deux éléments précités qui permettra d'obtenir un résultat dont la valeur est une indication de la quantité d'antibiotique présent.

Il convient de remarquer qu'en fonction de la méthode de détection choisie, l'agent de marquage peut être couplé soit à l'agent de reconnaissance soit à l'antibiotique ou à une substance analogue de l'antibiotique du point de vue de sa reconnaissance par l'agent de reconnaissance. Il existe également des procédés dans lesquels l'agent de reconnaissance ou l'antibiotique ou la substance analogue de l'antibiotique contient intrinsèquement l'agent de marquage (par exemple un antibiotique marqué radioactivement).

Le brevet US 4.239.852 décrit un procédé microbiologique pour la détection d'antibiotiques à noyau β-lactame dans le lait. Selon ce procédé, l'échantillon de lait est incubé, d'une part, en présence de parties de cellules d'un micro-organisme très sensible aux antibiotiques, en particulier Bacillus stearothermophilus, et, d'autre part, en présence d'un antibiotique marqué par un élément radioactif ou par une enzyme. L'incubation est menée dans des conditions permettant aux antibiotiques éventuellement présents dans l'échantillon et à l'antibiotique marqué de se lier aux parties de cellules.

Après incubation, les parties de cellules sont séparées du mélange, puis lavées. Ensuite, la quantité d'antibiotique marqué lié aux parties de cellules est déterminée et comparée à un standard. La quantité d'antibiotique marqué lié aux parties de cellules est inversement proportionnelle à la concentration d'antibiotique présent dans l'échantillon de lait analyse.

Ce procédé requiert des manipulations relativement délicates, notamment lors de la séparation des parties de cellules du mélange. En outre, dans sa version la plus sensible, permettant de détecter la pénicilline G jusqu'à 0,01 U.I./ml, voire même 0,001 U.I./ml de lait, ce procédé utilise un antibiotique marqué par un élément radioactif (¹⁴C ou ¹²⁵I). Dans ce cas, la détermination de la quantité d'antibiotique éventuellement présent dans le lait nécessite d'avoir recours à un appareil spécial, comme par exemple un compteur à scintillation. De plus, la manipulation de produits radioactifs, même en très faibles quantités n'est pas totalement exempte de danger pour la personne qui effectue l'analyse.

La demande de brevet européen 593.112 décrit une autre méthode permettant la détection d'antibiotiques dans le lait. Cette méthode utilise une protéine isolée à partir d'un microorganisme sensible aux antibiotiques, tel que Bacillus stearothermophilus. Cette protéine est en outre marquée par une enzyme telle qu'une peroxydase.

Le test procède de la manière suivante: un échantillon de lait est incubé dans un tube en présence de la protéine marquée; après incubation, le lait est transféré dans un second tube sur les parois duquel un antibiotique de référence a été immobilisé; on procède à une seconde incubation, puis le contenu du tube est éliminé; les parois de ce second tube sont lavées trois fois au moyen d'une solution de lavage, qui est elle-même éliminée, puis les résidus présents dans le second tube sont transférés sur un papier absorbant; on ajoute alors un substrat colorant dans le second tube qui est incubé une nouvelle fois, puis on ajoute une solution arrêtant le développement de la couleur; la coloration du tube est comparée à la coloration d'un test identique effectué parallèlement sur un échantillon standard d'antibiotique. La quantité de protéine marquée immobilisée sur le support, et donc, l'intensité de la coloration, est inversement proportionnelle à la quantité d'antibiotique présent dans l'échantillon de lait analysé.

Selon l'exemple 1 de cette demande de brevet, ce test permet de détecter la pénicilline G jusqu'à des concentrations de l'ordre de 5 ppb et permet la détection de l'amoxicilline (5 ppb), de l'ampicilline (10 ppb), de la cephapirine (5 ppb) et du ceftiofur (5 ppb). Ce test ne permet pas de détecter la pénicilline, l'amoxicilline et l'ampicilline aux normes fixées par les réglementations européennes et, par ailleurs, ce test est extrêmement complexe; il ne répond pas entièrement aux critères de sensibilité et de simplicité recherchés dans le contexte de la présente invention.

On connaît également d'autres types de procédés enzymatiques permettant de déterminer de faibles concentrations d'antibiotiques dans le lait (J.M. FRERE et al., Antimicrobial Agents and Chemotherapy, 18(4), 506-510 (1980) ainsi que les brevets EP 85.667 et EP 468.946) qui sont basés sur l'utilisation d'une enzyme spécifique, en l'occurrence, la D-alanyl-D-alanine-carboxypeptidase exocellulaire soluble, qui est produite par Actinomadura R39 (ci-après désignée "enzyme R39"). L'enzyme R39 possède une activité spécifique d'hydrolyse des groupements D-alanyl-D-alanine de divers peptides et est également capable d'hydrolyser des thioesters particuliers.

En outre, l'enzyme R39 réagit avec les antibiotiques à noyau β-lactame pour former très rapidement un complexe enzyme-antibiotique équimolaire, inactif et sensiblement irréversible.

Dans la version la plus récente de ce test (EP 468.946), un volume déterminé d'un échantillon du liquide à examiner est incubé avec une quantité prédéterminée de l'enzyme R39 dans des conditions permettant à l'antibiotique β-lactame éventuellement présent dans l'échantillon de réagir avec l'enzyme pour former un complexe enzyme-antibiotique équimolaire, inactif et sensiblement irréversible.

Ensuite, une quantité déterminée de substrat de type thioester est incubée avec le produit obtenu au premier stade dans des conditions permettant l'hydrolyse du substrat par l'enzyme R39 résiduelle qui n'a pas été complexée avec l'antibiotique lors de la première incubation. La quantité d'acide mercaptoalcanoïque ainsi formée est alors déterminée par dosage colorimétrique à l'aide d'un réactif capable de produire une coloration par réaction avec le groupe SH libre de l'acide mercaptoalcanoïque. L'intensité de la coloration est comparée à un étalon, préalablement établi à partir d'échantillons contenant des quantités connues d'antibiotiques. La détermination quantitative peut se faire par mesure au spectrophotomètre; dans le cas du lait, la clarification préalable de l'échantillon peut s'avérer nécessaire.

Selon les exemples de réalisation du brevet EP 468946, ce procédé permet de déterminer, dans le lait, 10 ppb de pénicilline G pour un temps total d'incubation de 5 minutes et de l'ordre de 2,5 ppb de pénicilline G pour un temps total d'incubation de 15 minutes.

Etant donné les critères de rapidité, de simplicité et de sensibilité exigés des méthodes de détection d'antibiotiques dans les produits alimentaires, la demanderesse s'est fixé comme objectif de rechercher de nouvelles méthodes encore plus performantes permettant la détection d'antibiotiques dans un liquide biologique. En particulier, la demanderesse s'est fixé comme objectif de rechercher des méthodes permettant de détecter en un seul test la majorité des antibiotiques dont la teneur est réglementée par les autorités européennes et américaines. De plus, les méthodes recherchées doivent permettre d'obtenir ce résultat en un nombre limite d'étapes, de préférence éxécutables par du personnel non qualifié. La demanderesse a également recherché des méthodes permettant d'atteindre ces objectifs avec un temps d'incubation réduit par rapport aux procédés existants.

La demanderesse vient de découvrir de nouveaux procédés pour la détection d'antibiotiques à noyau β-lactame dans un liquide biologique qui permettent d'atteindre de manière remarquable ces objectifs.

C'est pourquoi la présente invention se rapporte à de nouveaux procédés pour la détection d'antibiotiques à noyau β-lactame dans un liquide biologique comprenant les étapes suivantes
a) la mise en contact d'un volume déterminé dudit liquide biologique avec une quantité d'agent de reconnaissance et l'incubation du mélange ainsi obtenu dans des conditions permettant la complexation des antibiotiques éventuellement présents dans ledit liquide biologique avec l'agent de reconnaissance,
b) la mise en contact du mélange obtenu à l'étape a) avec au moins un antibiotique de référence immobilisé sur un support, dans des conditions permettant la complexation de l'antibiotique de référence avec la quantité d'agent de reconnaissance qui n'a pas réagi à l'étape a), et
c) la détermination de la quantité d'agent de reconnaissance fixée sur le support, caractérisés en ce que l'agent de reconnaissance comprend le récepteur BlaR ou le récepteur BlaR-CTD obtenu à partir de Bacillus licheniformis.

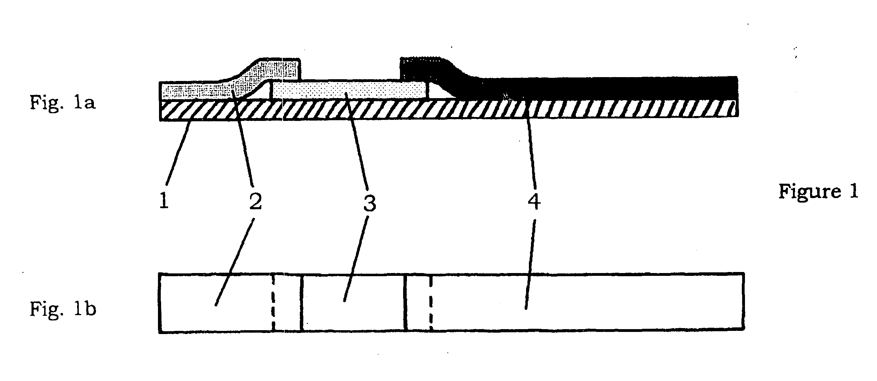

La Figure 1 illustre un type de support utilisable selon la présente invention, se présentant sous la forme d'un dispositif d'essai comprenant un support solide (1) sur lequel des membranes (2), (3) et (4) sont fixées. La Figure 1a présente une vue frontale et la Figure 1b présente une vue en section longitudinale du dispositif d'essai.

Les performances exceptionnelles du procédé selon la présente invention reposent sur l'utilisation d'un agent de reconnaissance particulier, qui comprend un récepteur sensible aux antibiotiques à noyau β-lactame obtenu à partir de Bacillus licheniformis, à savoir la protéine BlaR dont l'isolement et la séquence peptidique sont décrits dans Y. ZHU et al., J. Bacteriol., 1137-1141, (1990), ou le polypeptide BlaR-CTD qui est le domaine carboxy terminal de BlaR dont l'isolement et la séquence peptidique sont décrits dans B. JORIS et al., FEMS Microbiology Letters, 107-114, (1990).

L'utilisation des récepteurs BlaR ou BlaR-CTD selon la présente invention pour la détection d'antibiotiques à noyaux β-lactame présente des avantages importants par rapport aux agents de reconnaissance utilisés jusqu'à présent. En effet, les récepteurs BlaR et BlaR-CTD sont capables de complexer très rapidement un nombre très élevé d'antibiotiques, et ce, à une température d'incubation inférieure à celle nécessitée pour les agents de reconnaissance connus tels que, par exemple, les récepteurs obtenus à partir de Bacillus stearothermophilus.

A titre d'exemples d'antibiotiques détectables grâce aux procédés selon la présente invention, on peut citer les antibiotiques suivants : benzylpénicilline (ou pénicilline G), ampicilline, amoxicilline, carbénicilline, méthylcilline, cloxacilline, 6-APA, monolactame, aztréoname, mécilliname, céphalexine, céphaloglycine, céphaloridine, nitrocéphine, céfatoxime, céfuroxime, ceftiofur, céphapyrine, 7-ACA. Plus particulièrement, les procédés selon la présente invention permettent de détecter tous les antibiotiques contrôlés par les autorités américaines et européennes, et ce, jusqu'aux seuils limites tolérés.

Les procédés selon l'invention permettent de détecter les antibiotiques à noyau β-lactame dans les liquides biologiques tels que le lait, l'urine, le sang, le sérum, la salive, les extraits de viande, les liquides de fermentation ou les milieux aqueux tamponnés.

Selon un mode de mise en oeuvre préféré des procédés de l'invention, l'agent de reconnaissance est utilisé sous une forme couplée à un agent de marquage. Cet agent de marquage peut être de natures diverses. L'agent de marquage peut être de type particulaire, comme les particules colloïdales métalliques (platine, or, argent, ...), les particules colloïdales de sélénium, carbone, soufre ou tellure, ou encore les particules colloïdales de latex synthétiques colorés. L'agent de marquage peut également être une substance fluorescente, telles que la fluorescéine activée (disponible chez Boeringher-Mannheim Biochemica), l'isocyanate de fluorescéine, le tétraméthylisocyanate de rhodamine ou tout autre substance fluorescente connue de l'homme du métier. L'agent de marquage peut également être enzymatique, par exemple une β-lactamase, une peroxydase, une phosphatase, .... Dans ce cas, les récepteurs BlaR ou BlaR-CTD sont couplés par voie chimique ou génétique à cet agent de marquage enzymatique pour former une protéine de fusion.

Le couplage de l'agent de reconnaissance avec l'agent de marquage peut être effectué selon les méthodes conventionnelles connues de l'homme du métier. L'agent de reconnaissance peut être fixé soit directement sur l'agent de marquage, soit par la formation d'un complexe intermédiaire. Le couplage entre l'agent de reconnaissance et l'agent de marquage peut avoir lieu à différents moments lors de la mise en oeuvre des procédés de l'invention. Selon un premier mode de réalisation, le couplage entre l'agent de reconnaissance et l'agent de marquage a lieu avant la mise en contact de l'agent de reconnaissance et l'échantillon de liquide biologique à analyser. Selon d'autres modes de réalisation des procédés de l'invention, le couplage entre l'agent de reconnaissance et l'agent de marquage peut avoir lieu pendant ou après la mise en contact de l'agent de reconnaissance avec l'échantillon de liquide biologique. De préférence, le marquage de l'agent de reconnaissance a lieu avant la mise en contact de l'agent de reconnaissance avec l'échantillon à analyser.

La première étape a) des procédés selon l'invention consiste en la mise en contact d'un volume déterminé de liquide biologique avec une quantité d'agent de reconnaissance et l'incubation du mélange obtenu dans des conditions permettant la complexation des antibiotiques éventuellement présents dans le liquide biologique avec l'agent de reconnaissance.

L'incubation du liquide biologique avec le récepteur BlaR ou BlaR-CTD peut-être effectuée dans un intervalle de températures compris entre 4 et 60 °C. De préférence, cette température est de l'ordre de 47°C. Une augmentation de la température d'incubation aura pour effet une diminution de sa durée et inversement. Il est donc toujours possible de réduire la durée du procédé en augmentant la température.

Dans la seconde étape b) du procédé selon la présente invention, le mélange obtenu à l'étape a) est mis en contact avec au moins un antibiotique de référence immobilisé sur un support.

Les supports utilisables selon la présente invention peuvent être de types très variés. Il peut s'agir de supports solides tels que tubes, plaques ou tiges recouverts d'une préparation d'antibiotique de référence. Il peut s'agir d'un dispositif d'essai se présentant sous la forme d'un support solide sur lequel des membranes sont fixées, une ou plusieurs substances de capture étant placées dans une zone de détection déterminée. Il peut s'agir de supports se présentant sous la forme de billes (agarose, polystyrène, ...) magnétiques ou non, capables de former un gel et sur lesquelles l'antibiotique de référence est immobilisé.

L'immobilisation de l'antibiotique de référence sur le support peut se faire par les méthodes connues de l'homme du métier, par exemple par absorption covalente ou non-covalente sur le support, éventuellement par l'intermédiaire d'un espaceur.

Selon un mode de réalisation particulier de l'invention, les étapes a) et b) peuvent avoir lieu simultanément.

L'étape c) du procédé selon la présente invention consiste à déterminer les récepteurs qui se sont fixés sur le support sur lequel l'antibiotique de référence est immobilisé. La méthode utilisée pour cette détermination est directement liée au type d'agent de marquage utilisé. Si l'agent de marquage est enzymatique, l'étape de détermination fera appel à une réaction spécifique de cet enzyme associée, par exemple, à la production d'un coloration déterminée. Si l'agent de marquage est fluorescent, la détermination s'effectue simplement par mesure de la fluorescence du support. Dans le cas de particules métalliques ou de latex colorés, la présence de récepteurs fixés sur le support se traduit par une coloration dont l'intensité est directement proportionnelle au nombre de récepteurs fixés sur le support. Quel que soit le type d'agent de marquage utilisé, l'intensité du signal détecté est inversement proportionnelle à la quantité d'antibiotique présent dans l'échantillon analysé.

La présente invention concerne aussi des trousses d'essai pour la détection d'antibiotiques dans un liquide biologique comprenant au moins un agent de reconnaissance qui comprend un récepteur sensible aux antibiotiques à noyau β-lactame obtenu à partir de Bacillus licheniformis, et au moins un antibiotique de référence immobilisé sur un support, caractérisé en ce que l'agent de reconnaissance sensible aux antibiotiques à noyau β-lactame est le récepteur BlaR ou le récepteur BlaR-CTD obtenu à partir de Bacillus licheniformis.

Les exemples qui suivent illustrent différents aspects et modes d'implémentation de la présente invention sans toutefois en limiter sa portée.

### Exemple 1. Détermination d'antibiotiques à noyau β-lactame dans le lait.

Cet exemple illustre la détection dans le lait d'antibiotiques à noyau β-lactame contrôlés par les autorités sanitaires. Le test décrit dans cet exemple utilise le récepteur BlaR-CTD couplé à des billes d'or qui servent d'agents de marquage et utilise un support se présentant sous la forme d'un dispositif d'essai comprenant un support solide sur lequel des membranes sont fixées.

### 1.1. Couplage de BlaR-CTD aux billes d'or.

### 1.1.1. Biotinylation de BlaR-CTD.

3,79 ml d'une solution d'agent de reconnaissance BlaR-CTD à 6,6 mg/ml sont repris en tampon Phosphate de sodium 20 mM pH 7. On ajoute alors à cette solution de BlaR-CTD 41,71 ml de tampon bicarbonate (0,1 M en bicarbonate de sodium, pH 9) et 2 ml d'une solution d'ester 6-(biotinamido)caproïque de N-hydroxy-succinimide à 2,23 mg/ml également en tampon bicarbonate. Cette solution est agitée doucement sur agitateur pour tubes sur axe rotatif de type LABINCO (disponible chez VEL, Belgique) à une vitesse de 2 tours/minute pendant 2 heures à température ambiante et à l'abri de la lumière. 2,5 ml d'une solution de tampon Tris 1 M pH 8 sont incubés avec le mélange réactionnel dans les mêmes conditions pendant 30 minutes. La solution ainsi obtenue est dialysée contre du tampon HNM (Hepes 100mM, pH 8, NaCl 100 mM, MgCl₂ 50 mM) pendant 24 heures. De cette manière, on obtient une solution de BlaR-CTD biotinylé que l'on dilue dans du tampon HNM-BSA (Hepes 500 mM, pH 8, NaCl 500 mM, MgCl₂ 250 mM. BSA 10 mg/ml) jusqu'à une concentration de 250 µg de BlaR-CTD biotinylé par ml de tampon. Cette solution est stockée à -20 °C.

### 1.1.2. Agent de marquage.

Comme agent de marquage, on utilise des particules d'or ayant un diamètre de 40 nm, sur lesquelles un anticorps de chèvre anti-biotine a été déposé sous la forme de suspensions dans une solution aqueuse de tétraborate de sodium à 2 mM à pH 7,2, stabilisée par de l'azoture de sodium à 0,1% (disponible chez BRITISH BIOCELL (Réf. GAB40). La densité optique de ces suspensions à 520 nm est d'environ 10 et la concentration en protéine est d'environ 24 µg/ml.

### 1.1.3. Couplage de BlaR-CTD biotinylé aux billes d'or.

La solution de BlaR-CTD biotinylé préparée à l'exemple 1.1.1 est diluée 114,7 fois par du tampon HNM-BSA (Hepes 500 mM, pH 8, NaCl 500 mM, MgCl₂ 250 mM, BSA 10 mg/ml). On mélange à température ambiante 22,5 parties en volume de cette solution diluée de BlaR-CTD biotinylé, 7,5 parties en volume de tampon HNM-BSA, 9,27 parties en volume de suspension de particules d'or servant à marquer BlaR-CTD biotinylé et 6 parties en volume de suspension de particules d'or de référence (voir exemple 1.1.4 ci-dessous).

### 1.1.4. Référence indépendante.

Dans ce test, on utilise également une substance de référence qui fournit une bande dont l'intensité permet de quantifier rapidement la quantité d'antibiotique présent dans l'échantillon.

Pour cela, on utilise des particules d'or de 40 nm sur lesquelles un anticorps de chèvre anti-Immunoglobuline de lapin a été déposé. Ces particules sont disponibles chez BRITISH BIOCELL (Réf. GAR40), sous la forme de suspensions dans une solution aqueuse de tétraborate de sodium à 2 mM à pH 7,2, stabilisée par de l'azoture de sodium à 0,1%. La densité optique de ces suspensions à 520 nm est d'environ 3 et la concentration en protéine est d'environ 6 µg/ml.

### 1.2. Dispositif d'essai.

Le dispositif d'essai utilisé comprend un support solide (1) qui possède une première et une seconde extrémité, sur lequel sont fixées, successivement, en partant de la première extrémité,
- une membrane (2) permettant la purification du liquide analysé,
- une membrane (3) sur laquelle deux substances de capture (antibiotique de référence et substance capable de fixer la référence indépendante) sont immobilisées, et
- une membrane (4) absorbante

### 1.2.1. Assemblage du dispositif d'essai.

Des cartes ayant une taille de 300 x 76,2 mm sont d'abord assemblées au moyen d'un laminateur de type Chlamshell Laminator (disponible chez Bio Dot, Inc.) selon la méthode suivante:

On découpe un rectangle de support plastique de type ArCare 8565 (disponible chez Adhesive Research) de dimension 300 x 76,2 mm (support solide (1)). On découpe ensuite un rectangle de membrane Leukosorb LK4 (disponible chez Pall Gelman Sciences) de dimension 300 x 20 mm (membrane (2)), un rectangle de membrane Hi-Flow SX (disponible chez Millipore) de dimension 300 x 25 mm (membrane (3)), un rectangle de membrane de cellulose 3 mm (disponible chez Whatmann) de dimension 300 x 40 mm (membrane (4)).

On loge successivement les membranes (2) et (4), puis (3) dans un emplacement spécifique du moule inférieur du laminateur. Le support solide (1) recouvert d'adhésif est quant à lui maintenu dans le couvercle de l'appareil, le côté adhésif étant exposé à l'air. Les membranes logées dans le moule inférieur sont mises en contact avec le support adhésif en refermant le laminateur; les membranes sont maintenues exactement en place au moyen d'une succion d'air réalisée par une pompe à vide. Lorsque le vide est rompu, on récupère une carte constituée du support solide (1) sur lequel les membranes (2), (3) et (4) sont fixées.

On dépose ensuite les solutions suivantes sur la membrane (3): côté proximal: première substance de capture; bande n° 1; côté distal: deuxième substance de capture; bande n°2. Ces substances de capture sont déposées au moyen d'un "Dispenser" de type X-Y Platform Biojet Quanti-3000 de Bio Dot, Inc.

Les solutions déposées sont immédiatement évaporées en plaçant l'ensemble de la carte pendant une minute sous un air pulsé chaud à 60°C.

Les cartes obtenues après assemblage sont découpées en lamelles à l'aide d'un appareil de type guillotine ou à l'aide d'un appareil rotatif (disponible chez Bio Dot, Kinematic ou Akzo). Les lamelles des extrémités sont écartées, les autres lamelles étant prêtes à l'emploi.

La Figure 1 illustre un tel dispositif d'essai.

Pour leur conservation, les dispositifs d'essai sont placés dans un récipient opaque et hermétique en présence d'un agent dessicant (Air Sec, France).

### 1.2.2. Première substance de capture - Antibiotique de référence.

8 ml d'une solution contenant 213 mg de Gamma Globuline Humaine (G4386, Sigma) et 8,6 mg de chlorhydrate de 2-Iminothiolane (Aldrich, 33056-6) dans du tampon carbonate de sodium (100 mM, pH 9) sont incubés pendant une heure à 25°C.

Par ailleurs, 20 ml d'une solution contenant 119,8 mg de céphalosporine-C et 54 mg de sulfosuccinimidyl 4-(N-maléimidométhyl)cyclohexane-1-carboxylate (sSMCC, 22322 Pierce)dans du tampon carbonate de sodium (100 mM, pH 9) sont incubés pendant une heure à 25°C.

Les deux solutions préparées précédemment sont ensuite mélangées. On ajuste le pH de la solution résultante à 7,1 par ajout de 3 ml NaH₂PO₄ 500 mM et l'on incube pendant deux heures à 25°C. Le mélange obtenu après incubation est dialysé trois fois contre 1 litre de tampon phosphate de sodium (10 mM, pH 7,5). La solution résultante est filtrée sur un filtre à 0,22 µm, puis elle est aliquotée et congelée à -20°C jusqu'à utilisation.

Lors de l'utilisation, les aliquotes sont décongelées, et on y ajoute un colorant alimentaire avant d'effectuer le dépôt sur la membrane afin d'apprécier à tout moment la position exacte du dépôt et la qualité du trait.

La première substance de capture permet de fixer BlaR-CTD couplé aux billes d'or présent en excédent par rapport à la quantité d'antibiotique présent dans l'échantillon.

### 1.2.3. Deuxième substance de capture - Substance capable de fixer la référence indépendante.

Pour la deuxième substance de capture, on utilise une solution d'immunoglobuline de lapin (Sigma I 5006) à une concentration de 0,5 mg/ml en immunoglobuline dans du tampon phosphate de sodium 10 mM, pH 7,5, gamma globuline humaine 5 mg/ml. Cette seconde substance de capture arrête la référence indépendante lors de la migration du liquide sur le dispositif d'essai.

### 1.3. Détermination des antibiotiques dans le lait.

### 1.3.1. Test en 3 minutes - Test rapide

On prépare 7 échantillons de lait frais contenant respectivement 0;1;2;3;4;5 et 6 ppb de pénicilline G. Chacune de ces solutions est alors analysée de la manière suivante :

On prélève un aliquot de 200 µl d'échantillon de lait et 45.27 µl de solution préparée à l'exemple 1.1.3 que l'on place dans une fiole en verre. Ce mélange est incubé pendant 1 minute à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 1 ci-dessous reprend les résultats obtenus pour les 7 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Pénicilline G présente dans l'échantillon.

**Tableau 1**

| Pénicilline G | Intensité | |
|---|---|---|
| (ppb) | 1ère bande | 2ème bande |
| 0 | 10 | 6 |
| 1 | 9 | 6 |
| 2 | 9 | 6 |
| 3 | 4 | 6 |
| 4 | 0 | 6 |
| 5 | 0 | 6 |
| 6 | 0 | 6 |

Dans cet exemple, lorsque la première bande a une intensité inférieure à celle de la deuxième bande, le test est considéré comme positif. Les résultats présentés dans le Tableau 1 montrent que ce test permet de détecter en 3 minutes moins de 4 ppb de pénicilline G dans un échantillon de lait.

Des essais ont également été réalisés avec d'autres antibiotiques à noyau β-lactame dans les mêmes conditions. Ce test réalisé en 3 minutes permet de détecter l'amoxicilline jusqu'à 5 ppb, l'ampicilline jusqu'à 5 ppb, la cloxacilline à moins de 10 ppb, la dicloxacilline à moins de 20 ppb, l'oxacilline à moins de 20 ppb et la céphapirine jusqu'à 20 ppb dans un échantillon de lait.

### 1.3.2. Test en 5 minutes

On prépare 6 échantillons de lait frais contenant respectivement 0;2;4;6;8 et 10 ppb de cloxacilline. Chacune de ces solutions est alors analysée de la manière suivante.

On prélève un aliquot de 200 µl d'échantillon de lait et 45.27 µl de solution préparée à l'exemple 1.1.3 que l'on place dans une fiole en verre. Ce mélange est incubé pendant 3 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 2 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Cloxacilline présente dans l'échantillon.

**Tableau 2**

| Cloxacilline | Intensité | |
|---|---|---|
| (ppb) | 1ère bande | 2ème bande |
| 0 | 10 | 6 |
| 2 | 6 | 6 |
| 4 | 5 | 6 |
| 6 | 3 | 6 |
| 8 | 3 | 6 |
| 10 | 3 | 6 |

Dans cet exemple, lorsque la première bande a une intensité inférieure à celle de la deuxième bande, le test est considéré comme positif. Les résultats présentés dans le Tableau 2 montrent que ce test permet de détecter en 5 minutes jusqu'à 4 ppb de cloxacilline dans un échantillon de lait.

Des essais ont également été réalisés avec d'autres antibiotiques à noyau β-lactame dans les mêmes conditions. Ce test réalisé en 5 minutes permet de détecter la pénicilline G jusqu'à 3 ppb, l'amoxicilline jusqu'à 4 ppb, l'ampicilline jusqu'à 4 ppb, la dicloxacilline jusqu'à 8 ppb, l'oxacilline jusqu'à 8 ppb, la céphapirine jusqu'à 16 ppb, le ceftiofur jusqu'à 100 ppb, la cefquinone à moins de 20 ppb, la nafcilline jusqu'à 20 ppb et la céfazoline jusqu'à 60 ppb dans un échantillon de lait.

Ce test convient particulièrement bien comme test de tri avant le dépotage des camions de lait dans les silos.

### 1.3.3. Test en 9 minutes

On prépare 6 échantillons de lait frais contenant respectivement 0;4;6;8;10 et 12 ppb de céphapirine. Chacune de ces solutions est alors analysée de la manière suivante.

On prélève un aliquot de 200 µl d'échantillon de lait et 45.27 µl de solution préparée à l'exemple 1.1.3 que l'on place dans une fiole en verre. Ce mélange est incubé pendant 7 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 3 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Céphapirine présente dans l'échantillon.

**Tableau 3**

| Céphapirine | Intensité | |
|---|---|---|
| (ppb) | 1ère bande | 2ème bande |
| 0 | 10 | 6 |
| 4 | 6 | 6 |
| 6 | 5 | 6 |
| 8 | 4 | 6 |
| 10 | 3 | 6 |
| 12 | 3 | 6 |

Dans cet exemple, lorsque la première bande a une intensité inférieure à celle de la deuxième bande, le test est considéré comme positif. Les résultats présentés dans le Tableau 3 montrent que ce test permet de détecter en 9 minutes jusqu'à 6 ppb de céphapirine dans un échantillon de lait.

Des essais ont également été réalisés avec d'autres antibiotiques à noyau β-lactame dans les mêmes conditions. Ce test réalisé en 9 minutes permet de détecter la pénicilline G jusqu'à 3 ppb, l'amoxicilline jusqu'à 4 ppb, l'ampicilline jusqu'à 4 ppb, la cloxacilline jusqu'à 4 ppb, la dicloxacilline à moins de 8 ppb, l'oxacilline à moins de 8 ppb, le ceftiofur jusqu'à 80 ppb, la cefquinone à moins de 20 ppb, la nafcilline à moins de 20 ppb et la céfazoline jusqu'à 45 ppb dans un échantillon de lait.

Ce test réalisé en 9 minutes permet donc de détecter tous les antibiotiques contrôlés actuellement par les autorités européennes, et ce, jusqu'aux limites légales imposées par ces autorités

### 1.3.4. Test en 20 minutes

On prépare 6 échantillons de lait frais contenant respectivement 0;20;30;40;50 et 60 ppb de ceftiofur. Chacune de ces solutions est alors analysée de la manière suivante.

On prélève un aliquot de 200 µl d'échantillon de lait et 45.27 µl de solution préparée à l'exemple 1.1.3 que l'on place dans une fiole en verre. Ce mélange est incubé pendant 18 minutes à 47°C. On prend un dispositif d'essai que l'on place verticalement dans la fiole en verre de manière à ce que la première extrémité du dispositif d'essai soit en contact avec le mélange et de manière à ce que la seconde extrémité repose sur la paroi de la fiole en verre. On laisse migrer le mélange sur le dispositif d'essai tout en incubant l'ensemble pendant 2 minutes à 47°C.

Le Tableau 4 ci-dessous reprend les résultats obtenus pour les 6 échantillons testés. Une valeur d'intensité allant de 0 à 10 est attribuée aux bandes détectées, la valeur 10 étant donnée pour la bande la plus intense et la valeur 0 étant donnée pour la bande la moins intense. Selon cette échelle, on attribue une valeur de 6 à la bande de référence. L'intensité du signal observé dans la première bande est inversement proportionnelle à la quantité de Ceftiofur présente dans l'échantillon.

**Tableau 4**

| Ceftiofur | Intensité | |
|---|---|---|
| (ppb) | 1ère bande | 2ème bande |
| 0 | 10 | 6 |
| 20 | 6 | 6 |
| 30 | 5 | 6 |
| 40 | 4 | 6 |
| 50 | 3 | 6 |
| 60 | 3 | 6 |

Dans cet exemple, lorsque la première bande a une intensité inférieure à celle de la deuxième bande, le test est considéré comme positif. Les résultats présentés dans le Tableau 4 montrent que ce test permet de détecter en 20 minutes jusqu'à 30 ppb de ceftiofur dans un échantillon de lait.

Ce test en 20 minutes permet donc de détecter en un seul test tous les antibiotiques contrôlés actuellement par les autorités européennes et américaines, et ce, jusqu'aux limites légales imposées par ces autorités.

### Exemple 2. Détermination de 6 antibiotiques dans le lait.

Cet exemple illustre la détection dans le lait des 6 antibiotiques à noyau β-lactame contrôlés par les autorités américaines. Le test décrit dans cet exemple utilise le récepteur BlaR-CTD sous la forme d'une protéine de fusion avec la β-lactamase et utilise un support se présentant sous la forme de billes magnétiques.

### 2.1. Protéine de fusion BlaR-CTD- β-lactamase.

La protéine de fusion BlaR-CTD- β-lactamase est obtenue par couplage génétique entre le récepteur BlaR-CTD (B. JORIS et al., FEMS Microbiology Letters, 107-114, 1990) et la β-lactamase à Zn de Bacillus cereus (M. Hussain et al., 1985, J. Bact., 164:1, 223-229, 1985).

Le couplage est réalisé de la manière suivante:

2.1.1. Construction du plasmide: un couplage 1/1 a été réalisé entre les gènes du polypeptide BlaR-CTD et de la β-lactamase: le gène codant pour la β-lactamase a été introduit en phase et derrière le gène de BlaR-CTD. Le plasmide porteur de la fusion génétique présente la résistance à la kanamycine. La protéine de fusion est ci-après dénommée Fus 1.

### 2.1.2. Production:

- Souche: le plasmide portant les gène fusionnés a été introduit dans E. Coli. Les clones porteurs du plasmide recombinant sont sélectionnés sur LB + Km (50 µg/ml).
- Sélection: le marquage de l'extrait cellulaire avec un antibiotique radioactif, suivi d'une électrophorèse sur gel de polyacrylamide dénaturant, montre que la majeure partie de la protéine est produite sous forme d'un produit de fusion dont la masse molaire est d'environ 50.000. Cependant, une protéolyse post-traductionnelle semble dissocier un très faible pourcentage (2%) de ces molécules en deux activités distinctes.
- Culture: 500 ml de milieu LB + Km (50 µg/ml) sont inoculés à partir de cellules recombinantes conservées à -70°C. La préculture est incubée à 37°C et agitée une nuit à 225 tours par minute. 18 litres de milieu LB + Km (50 µg/ml) sont inoculés avec 500 ml de cette préculture dont la densité optique à 600 nm est de 4. La culture de 18 litres est arrêtée lorsque la densité optique atteint la valeur de 6.

2.1.3. Extraction: Immédiatement après arrêt de la culture, les cellules sont filtrées puis centrifugées. Le surnageant du culot cellulaire lysé au désintégrateur est gardé. Il contient la protéine de fusion FUS1.

### 2.1.4. Purification:

- Tampons utilisé:
   - Tampon A: Tris 20 mM pH 8,0, éthylène glycol 10%, DTT 50 µM;
   - Tampon B: tampon A + NaCl 1M.

La protéine de fusion est partiellement purifiée par chromatographie ionique et sur tamis moléculaire. Après dépôt de l'extrait et lavage sur une colonne QSFF (Pharmacia, Upsala) en tampon A, FUS1 est éluée par un gradient linéaire en tampon B. Les fractions actives (± 0,25 M en NaCl) sont ensuite rassemblées et déposée sur tamis moléculaire G-100 (Pharmacia, Upsala); elles sont éluées en tampon A. L'activité spécifique moyenne du lot récupéré est estimée à 30%. 2.1.5. Inhibition de la β-lactamase: la protéine de fusion (9/ 10 volume) est incubée en EDTA 50 mM (1/10 volume) pendant 45 minutes à 37°C. On vérifie l'inhibition à l'aide de nitrocéfine en tampon cacodylate 10 mM pH 6,0. En présence ou absence de Zn, le signal doit respectivement être positif ou négatif. Après inhibition, la concentration effective, mesurée sur base de l'activité β-lactamasique est de 7,74 pmole/µl.

### 2.2. Support solide: billes magnétiques - céphalosporine C (antibiotique de référence).

On utilise des particules BioMag 4100 (disponibles chez DRG Instrument Gmbh, Marburg, Germany, sous la référence AM 4100 B.) dont les extrémités NH₂ ont été activées au glutaraldéhyde de la manière suivante:
- un volume de solution initiale de particules BioMag 4100 est rincé 4 fois à l'aide de 5 volumes de tampon pyridine 0,01M pH 6,0. Les particules sont reprises dans 2,5 volumes de glutaraldéhyde à 5% en tampon pyridine et sont agitées par rotation pendant trois heures à température ambiante. Elles sont ensuite rincées 10 fois à l'aide de deux volumes de tampon Kpi 0,01 M à pH 7,0. Les particules sont ensuite remises en suspension dans un volume de céphalosporine C à 0,1 M en tampon Kpi et agitées par rotation pendant une nuit à +4°C. Le rinçage final se fait jusqu'à élimination complète de la céphalosporine C dans le tampon Kpi 0,1 M pH 7,0.

2.3. Détermination des 6 antibiotiques pénicilline G, ampicilline, amoxicilline, cloxacilline, cephapirine et ceftiofur dans le lait.

### 2.3.1. Solutions utilisées:

- solution 1: 700 picomoles de protéine de fusion lyophilisée en Tris 100 mM, pH 8, BSA 1 mg/ml, EDTA 50 mM, DTT 50 µM sont réhydratés avec 5 ml d'eau Milli-Q; 50 µl de cette solution sont nécessaires pour réaliser une mesure.
- solution 2: 2 ml de particules BioMag-céphalosporine C préparées à l'exemple 1.2 dans isopropanol à 100%; 20 µl sont nécessaires pour réaliser une mesure.
- solution 3: tampon cacodylate 10 mM, pH 6, NaCl 1M lyophilisé est réhydraté avec 500 ml d'eau Milli-Q.
- solution 4: 400 ml de nitrocéfine 10 mM en DMF sont dilués à 40 ml dans la solution 3; 400 µl sont nécessaires pour une détection.

### 2.3.2. Procédé de détection:

50 µl de la solution 1 sont placés en présence de 500 ml d'échantillons de lait dopés et incubés à 47°C pendant 2 minutes. 20 µl de la solution 2 sont mis en suspension dans le lait qui est de nouveau incubé pendant 2 minutes à 47°C. Les particules sont attirées contre la paroi du récipient à l'aide d'un aimant paramagnétique pendant que le surnageant est vidé du tube. Les particules sont rincées à deux reprises avec la solution 3 en procédant de manière similaire avec l'aimant. Enfin, 400 µl de la solution 4 sont incubés en présence des particules pendant 3 minutes à 47°C. On mesure alors l'absorbance de la solution résiduelle à 482 nm par rapport à la solution de nitrocéfine.

Ce procédé permet de détecter les 6 antibiotiques repris dans la réglementation américaine à des concentrations inférieures aux normes imposées par les autorités, à savoir, la pénicilline à 5 ppb, l'ampicilline à 10 ppb, l'amoxicilline à 10 ppb, la cloxacilline à 10 ppb, la céphapirine à 20 ppb et le ceftiofur à 50 ppb.

### Exemple 3. Détermination de 3 antibiotiques (pénicilline G, cloxacilline, ceftiofur) dans le lait.

Cet exemple illustre la détection dans le lait de 3 antibiotiques à noyau β-lactame contrôlés par les autorités sanitaires. Le test décrit dans cet exemple utilise le récepteur BlaR-CTD sous la forme de deux protéines de fusion avec la phosphatase alcaline et la peroxydase, respectivement, et utilise un support se présentant sous la forme d'une microplaque.

### 3.1. Protéine de fusion BlaR-CTD-phosphatase alcaline.

La protéine de fusion BlaR-CTD-phosphatase alcaline est obtenue par couplage chimique entre le récepteur BlaR-CTD (B. JORIS et al., FEMS Microbiology Letters, 107-114, (1990)) et la phosphatase alcaline activée disponible chez Boehringer Mannheim Biochemica sous la référence 1464752.

Le couplage est réalisé de la manière suivante:
3.1.1. Conjugaison: BlaR-CTD et la phosphatase alcaline sont dialysées dans du tampon carbonate/hydrogénocarbonate de sodium 100 mM à pH 9,8. 15 nanomoles de BlaR-CTD sont incubées en présence de 100 µl de phosphatase alcaline activée (20 mg/ ml) pendant deux heures à 25°C.
3.1.2. Arrêt de la réaction: On ajoute 40 µl d'une solution de triéthanolamine à 2mM, pH 8, puis 50 µl d'une solution de borohydrure de sodium à 200 mM. Le mélange est incubé pendant 30 min. à +4°C. On ajoute alors 25 µl d'une solution de triéthanolamine à 2 mM, pH 8, puis on incube à nouveau le mélange pendant 2 heures à + 4°C.
3.1.3. Stabilisation du couplage: on ajoute 10 µl d'une solution de glycine à 1M, pH 7,0.
3.1.4. Transfert dans le tampon de stockage: Le mélange réactionnel (environ 300 µl) est dialysé trois fois 8 heures contre 0,5 litre de tampon triéthanolamine 50 mM, pH 7,6, NaCl 150 mM, MgCl₂ 1 mM, ZnCl₂ 0,5 mM, glycine 10 mM à +4°C.
3.1.5. Titre final: le titre final du couplage est de l'ordre de 50 pmole de BlaR-CTD actif par µl de solution.

### 3.2. Protéine de fusion BlaR-CTD-peroxydase

La protéine de fusion BlaR-CTD-peroxydase est obtenue par couplage chimique entre le récepteur BlaR-CTD (B. JORIS et al., FEMS Microbiology Letters, 107-114, (1990)) et la peroxydase activée disponible chez Boehringer Mannheim Biochemica sous la référence 1428861.

Le couplage est réalisé de la manière suivante:
3.2.1. Conjugaison: BlaR-CTD et la peroxydase sont dialysées dans du tampon carbonate/hydrogénocarbonate de sodium 100 mM à pH 9,8. 40 nanomoles de BlaR-CTD sont incubées en présence de 100 µl de peroxydase activée (16 mg/ml) pendant deux heures à 25°C.
3.2.2. Arrêt de la réaction: On ajoute 40 µl d'une solution de triéthanolamine à 2mM, pH 8, puis 50 µl d'une solution de borohydrure de sodium à 200 mM. Le; mélange est incubé pendant 30 min. à +4°C. On ajoute alors 25 µl d'une solution de triéthanolamine à 2 mM, pH 8, puis on incube à nouveau le mélange pendant 2 heures à + 4°C.
3.2.3. Stabilisation du couplage: on ajoute 10 µl d'une solution de glycine à 1M, pH 7,0.
3.2.4. Transfert dans le tampon de stockage: Le mélange réactionnel (environ 400 µl) est dialysé trois fois 8 heures contre 0,5 litre de tampon phosphate de potassium 10 mM, pH 7,5, NaCl 200 mM, glycine 10 mM à +4°C.
3.2.5. Titre final: le titre final du couplage est de l'ordre de 100 pmole de BlaR-CTD actif par µl de solution.

### 3.3. Support solide: microplaque-céphalosporine C.

### 3.3.1. Préparation de la solution d'antibiotique de référence.

8 ml d'une solution contenant 213 mg de Gamma Globuline Humaine (G4386, Sigma) et 8,6 mg de chlorhydrate de 2-Iminothiolane (Aldrich, 33056-6) dans du tampon carbonate de sodium (100 mM, pH 9) sont incubés pendant une heure à 25°C.

Par ailleurs, 20 ml d'une solution contenant 119,8 mg de céphalosporine-C et 54 mg de sulfosuccinimidyl 4-(N-maléimidométhyl)cyclohexane-1-carboxylate (sSMCC, 22322 Pierce) dans du tampon carbonate de sodium (100 mM, pH 9) sont incubés pendant une heure à 25°C.

Les deux solutions préparées précédemment sont ensuite mélangées. On ajuste le pH de la solution résultante à 7,1 par ajout de 3 ml NaH₂PO₄ 500 mM et l'on incube pendant deux heures à 25°C. Le mélange obtenu après incubation est dialysé trois fois contre 1 litre de tampon phosphate de sodium (10 mM, pH 7,5). La solution résultante est filtrée sur un filtre à 0,22 µm.

### 3.3.2. Recouvrement des microplaques par l'antibiotique de référence.

On utilise des microplaques en polystyrène à haute adsorption protéique de la marque NUNK (Immuno Plate: type Maxisorp) ou de la marque GREINER (en microlon 600, référence 705071). Les cuvettes des microplaques sont lavées avec du tampon PBS 150 mM, pH 7,2. Ensuite un aliquot de la solution préparée à l'exemple 3.3.1. est incubé pendant 24 heures à 4°C dans les cuvettes. Après incubation, les cuvettes sont lavées trois fois avec du tampon PBS 150 mM, pH 7,2, Tween-20 0,1%. Le tube est alors saturé pendant deux heures à 20°C avec du tampon de saturation PBS 150 mM, pH 7,2, BSA 5%. Après trois lavages avec du tampon de lavage , les tubes sont séchés et stockés à 4°C à l'abri de l'humidité.

Pour les cuvettes destinées à l'agent de reconnaissance BlaR-CTD-phosphatase alcaline, le tampon de lavage utilisé est la diéthanolamine 1 M, pH 9,8, MgCl₂ 0,5 mM; pour les cuvettes destinées à l'agent de reconnaissance BlaR-CTD-peroxydase, le tampon de lavage utilisé est le phosphate de potassium 50 mM, pH 5.

### 3.4. Détermination de trois antibiotiques dans le lait.

1,4 picomoles d'agent de reconnaissance marqué sont incubés en présence de 100 µl de lait dopé pendant 5 minutes à 47°C. Le lait est transvasé au moyen d'une pipette dans une cuvette préalablement traitée comme indiqué à l'exemple 2.3. Le lait est alors incubé pendant 2 minutes à 47°C. Après élimination du lait, suivie de deux lavages par du tampon de lavage (cfr. exemple 2.3.2.), 300 µl de tampon contenant le substrat révélateur est incubé pendant 2 minutes dans la cuvette (substrat révélateur pour l'agent de reconnaissance BlaR-CTD-peroxydase: phosphate de potassium 50 mM, pH 5, ABTS 9,1 mM, H₂O₂ 0,002%; substrat révélateur pour l'agent de reconnaissance BlaR-CTD-phosphatase alcaline: diéthanolamine 1 M, pH 9,8, MgCl₂ 0,5 mM, 4-NPP 10 mM). La plaque est alors placée dans un spectrophotomètre automatique pour plaque ELISA, la longueur d'onde étant fixée à 405 nm.

Ce test permet de détecter les trois antibiotiques pénicilline G, cloxacilline et ceftiofur aux seuils de contrôle exigés par les autorités américaines (pénicilline G à 5 ppb, cloxacilline à 10 ppb et ceftiofur à 50 ppb).

### Exemple 4. Détermination des 6 antibiotiques pénicilline G, ampicilline, amoxicilline, cloxacilline, céphapirine et ceftiofur dans le lait.

Cet exemple illustre la détection dans le lait des 6 antibiotiques à noyau β-lactame contrôlés par les autorités américaines, jusqu'aux normes exigées actuellement par ces autorités. Le test décrit dans cet exemple utilise le récepteur BlaR-CTD sous la forme d'une protéine de fusion avec la phosphatase alcaline ou la peroxydase, et utilise un support se présentant sous la forme d'un tube recouvert.

### 4.1. Protéine de fusion BlaR-CTD-phosphatase alcaline.

Voir exemple 3.1.

### 4.2. Support solide: tube recouvert par un antibiotique de référence.

Dans cet exemple, on utilise des tubes en polystyrène à haute adsorption protéique de la marque NUNK (type Maxisorp) traités par une solution d'antibiotique de référence comme indiqué à l'exemple 3.3.2.

### 4.3. Détermination des 6 antibiotiques dans le lait.

7 picomoles d'agent de reconnaissance sont incubés en présence de 500 µl de lait pendant 5 minutes à 47°C dans un tube Eppendorf. Le lait est alors transvasé au moyen d'une pipette dans un tube traité comme expliqué à l'exemple 3.2. Le lait est alors incubé pendant 2 minutes à 47°C. Après élimination du lait, le tube est lavé deux fois par 1 ml de tampon diéthanolamine 1 M, pH 9,8, MgCl₂ 0,5 mM. On ajoute alors 500 µl de tampon contenant le substrat révélateur diéthanolamine 1 M, pH 9,8, MgCl₂ 0,5 mM, 4-NPP 10 mM et le substrat est incubé pendant 2 minutes à 47°C. On mesure ensuite l'absorbance du surnageant au moyen d'un spectrophotomètre dont la longueur d'onde est fixée à 405 nm.

Cette méthode permet de déterminer les 6 antibiotiques jusqu'aux normes exigées par les autorités américaines: pénicilline G à moins de 5 ppb; ampicilline à moins de 10 ppb; amoxicilline à moins de 10 ppb; cloxacilline à moins de 10 ppb; céphapirine à moins de 20 ppb; ceftiofur à moins de 50 ppb.

## Revendications

1. Procédé pour la détection d'antibiotiques à noyau β-lactame dans un liquide biologique comprenant les étapes suivantes
a) la mise en contact d'un volume déterminé dudit liquide biologique avec une quantité d'agent de reconnaissance et l'incubation du mélange ainsi obtenu dans des conditions permettant la complexation des antibiotiques éventuellement présents dans ledit liquide biologique avec l'agent de reconnaissance,
b) la mise en contact du mélange obtenu à l'étape a) avec au moins un antibiotique de référence immobilisé sur un support, dans des conditions permettant la complexation de l'antibiotique de référence avec la quantité d'agent de reconnaissance qui n'a pas réagi à l'étape a), et
c) la détermination de la quantité d'agent de reconnaissance fixée sur le support,
**caractérisé en ce que** l'agent de reconnaissance comprend le récepteur BlaR ou le récepteur BlaR-CTD obtenu à partir de Bacillus licheniformis.

2. Procédé selon la revendication 1, **caractérisé en ce que** le récepteur BlaR ou le récepteur BlaR-CTD est couplé à un agent de marquage choisi parmi les particules colloïdales métalliques, les particules colloïdales de sélénium, carbone, soufre ou tellure, les particules colloïdales de latex synthétiques colorés.

3. Procédé selon la revendication 1, **caractérisé en ce que** le récepteur BlaR ou le récepteur BlaR-CTD est couplé à un agent de marquage choisi parmi les substances fluorescentes.

4. Procédé selon la revendication 1, **caractérisé en ce que** le récepteur BlaR ou le récepteur BlaR-CTD est couplé à un agent de marquage choisi parmi les enzymes, tels que phosphatase alcaline, peroxydases, β-lactamases.

5. Procédé selon la revendication 4, **caractérisé en ce que** le récepteur BlaR ou le récepteur BlaR-CTD est couplé à l'agent de marquage enzymatique par voie chimique ou génétique.

6. Procédé selon l'une quelconque des revendications 2 à 5, **caractérisé en ce que** le couplage du récepteur BlaR ou du récepteur BlaR-CTD à l'agent de marquage a lieu avant l'étape a).

7. Procédé selon l'une quelconque des revendications 2 à 5, **caractérisé en ce que** le couplage du récepteur BlaR ou du récepteur BlaR-CTD à l'agent de marquage a lieu pendant ou après l'étape a).

8. Procédé selon l'une quelconque des revendications 1 à 7, **caractérisé en ce que** les étapes a) ou b) ont lieu simultanément.

9. Procédé selon l'une quelconque des revendications 1 à 8, **caractérisé en ce que** le support utilisé à l'étape b) est choisi parmi les tubes, plaques ou tiges recouverts d'un antibiotique de référence.

10. Procédé selon l'une quelconque des revendications 1 à 8, **caractérisé en ce que** le support utilisé à l'étape b) est un dispositif d'essai comprenant un support solide (1) qui possède une première et une seconde extrémité, sur lequel sont fixées, successivement, en partant de la première extrémité,
- une membrane (2) permettant la purification du liquide analysé,
- une membrane (3) sur laquelle une ou plusieurs substances de capture sont immobilisées, et
- une membrane (4) absorbante.

11. Procédé selon l'une quelconque des revendications de 1 à 8, **caractérisé en ce que** le support utilisé à l'étape b) est constitué d'un ensemble de billes magnétiques ou non.

12. Trousse d'essai pour la détection d'antibiotiques dans un liquide biologique par le procédé selon l'une quelconque des revendications 1 à 11, comprenant au moins un agent de reconnaissance sensible aux antibiotiques à noyau β-lactame obtenu à partir de Bacillus licheniformis, et au moins un antibiotique de référence immobilisé sur un support, **caractérisé en ce que** l'agent de reconnaissance sensible aux antibiotiques à noyau β-lactame est le récepteur BlaR ou le récepteur BlaR-CTD obtenu à partir de Bacillus licheniformis.

## Claims

1. Method of detecting β-lactam antibiotics in a biological fluid, comprising the following steps:
a) bringing of a given volume of said biological fluid into contact with an amount of recognition agent, and incubation of the resulting mixture under conditions that allow any antibiotics present in said biological fluid to complex with the recognition agent,
b) bringing of the mixture obtained in step a) into contact with at least one reference antibiotic immobilized on a support, under conditions that allow the reference antibiotic to complex with the amount of recognition agent that has not reacted in step a), and
c) determination of the amount of recognition agent fixed to the support,
**characterized in that** the recognition agent comprises the BlaR receptor or the BlaR-CTD receptor obtained from Bacillus licheniformis.

2. Method according to Claim 1, **characterized in that** the BlaR receptor or the BlaR-CTD receptor is coupled to a labelling agent selected from colloidal particles of a metal, colloidal particles of selenium, carbon, sulfur or tellurium, and colloidal particles of coloured synthetic latices.

3. Method according to Claim 1, **characterized in that** the BlaR receptor or the BlaR-CTD receptor is coupled to a labelling agent selected from fluorescent substances.

4. Method according to Claim 1, **characterized in that** the BlaR receptor or the BlaR-CTD receptor is coupled to a labelling agent selected from enzymes such as alkaline phosphatase, peroxidases and β-lactamases.

5. Method according to Claim 4, **characterized in that** the BlaR receptor or the BlaR-CTD receptor is chemically or genetically coupled to the enzymatic labelling agent.

6. Method according to any one of Claims 2 to 5, **characterized in that** the coupling of the BlaR receptor or the BlaR-CTD receptor to the labelling agent takes place before step a).

7. Method according to any one of Claims 2 to 5, **characterized in that** the coupling of the BlaR receptor or the BlaR-CTD receptor to the labelling agent takes place during or after step a).

8. Method according to any one of Claims 1 to 7, **characterized in that** steps a) and b) take place simultaneously.

9. Method according to any one of Claims 1 to 8, **characterized in that** the support used in step b) is selected from tubes, plates and rods coated with a reference antibiotic.

10. Method according to any one of Claims 1 to 8, **characterized in that** the support used in step b) is a test device comprising a solid support (1) with a first and second end, to which the following are fixed in succession, starting from the first end:
- a membrane (2) for purifying the fluid analysed,
- a membrane (3) on which one or more capturing substances are immobilized, and
- an absorbent membrane (4).

11. Process according to any one of Claims 1 to 8, **characterized in that** the support used in step b) consists of a number of magnetic or non-magnetic beads.

12. Test kit for the detection of antibiotics in a biological fluid by the method according to any one of Claims 1 to 11, comprising at least one recognition agent sensitive to β-lactam antibiotics and obtained from Bacillus licheniformis, and at least one reference antibiotic immobilized on a support, **characterized in that** the recognition agent sensitive to β-lactam antibiotics is the BlaR receptor or the BlaR-CTD receptor obtained from Bacillus licheniformis.

## Patentansprüche

1. Verfahren zum Nachweis von Antibiotika mit β-Lactamkern in einer biologischen Flüssigkeit, das die folgenden Schritte umfasst
a) das Inkontaktbringen eines bestimmten Volumens besagter biologischer Flüssigkeit mit einer Menge an Erkennungsmittel und die Inkubation des so erhaltenen Gemischs unter Bedingungen, die die Komplexierung der gegebenenfalls in besagter biologischer Flüssigkeit vorhandenen Antibiotika mit dem Erkennungsmittel ermöglichen,
b) das Inkontaktbringen des im Schritt a) erhaltenen Gemischs mit wenigstens einem auf einem Träger immobilisierten Referenzantibiotikum unter Bedingungen, die die Komplexierung des Referenzantibiotikums mit der Menge an im Schritt a) nicht umgesetztem Erkennungsmittel ermöglichen, und
c) die Bestimmung der auf dem Träger fixierten Menge an Erkennungsmittel,
**dadurch gekennzeichnet, dass** das Erkennungsmittel den Rezeptor BlaR oder den Rezeptor BlaR-CTD, erhalten aus Bacillus licheniformis, umfasst.

2. Verfahren gemäß Anspruch 1, **dadurch gekennzeichnet, dass** der Rezeptor BlaR oder der Rezeptor BlaR-CTD an ein Markierungsmittel gekoppelt ist, das unter den kolloidalen Metallpartikeln, den kolloidalen Selen-, Kohlenstoff-, Schwefel- oder Tellurpartikeln, den kolloidalen Partikeln von farbigen synthetischen Latizes ausgewählt ist.

3. Verfahren gemäß Anspruch 1, **dadurch gekennzeichnet, dass** der Rezeptor BlaR oder der Rezeptor BlaR-CTD an ein Markierungsmittel gekoppelt ist, das unter den fluoreszierenden Substanzen ausgewählt ist.

4. Verfahren gemäß Anspruch 1, **dadurch gekennzeichnet, dass** der Rezeptor BlaR oder der Rezeptor BlaR-CTD an ein Markierungsmittel gekoppelt ist, das unter den Enzymen, wie alkalische Phosphatase, Peroxidasen, β-Lactamasen, ausgewählt ist.

5. Verfahren gemäß Anspruch 4, **dadurch gekennzeichnet, dass** der Rezeptor BlaR oder der Rezeptor BlaR-CTD chemisch oder genetisch an das enzymatische Markierungsmittel gekoppelt ist.

6. Verfahren gemäß einem der Ansprüche 2 bis 5, **dadurch gekennzeichnet, dass** die Kopplung des Rezeptors BlaR oder des Rezeptors BlaR-CTD an das Markierungsmittel vor dem Schritt a) stattfindet.

7. Verfahren gemäß einem der Ansprüche 2 bis 5, **dadurch gekennzeichnet, dass** die Kopplung des Rezeptors BlaR oder des Rezeptors BlaR-CTD an das Markierungsmittel während oder nach dem Schritt a) stattfindet.

8. Verfahren gemäß einem der Ansprüche 1 bis 7, **dadurch gekennzeichnet, dass** die Schritte a) oder b) gleichzeitig stattfinden.

9. Verfahren gemäß einem der Ansprüche 1 bis 8, **dadurch gekennzeichnet, dass** der im Schritt b) verwendete Träger unter den mit einem Referenzantibiotikum überzogenen Röhrchen, Platten oder Stäbchen ausgewählt ist.

10. Verfahren gemäß einem der Ansprüche 1 bis 8, **dadurch gekennzeichnet, dass** der im Schritt b) verwendete Träger eine Testvorrichtung ist, die einen festen Träger (1) umfasst, der ein erstes und ein zweites Ende besitzt, auf dem, ausgehend vom ersten Ende, nacheinander fixiert sind
- eine Membran (2), die die Reinigung der analysierten Flüssigkeit ermöglicht,
- eine Membran (3), auf der eine oder mehrere Fangsubstanzen immobilisiert sind, und
- eine absorbierende Membran (4).

11. Verfahren gemäß einem der Ansprüche 1 bis 8, **dadurch gekennzeichnet, dass** der im Schritt b) verwendete Träger aus einer Gesamtheit magnetischer oder nicht magnetischer Kügelchen besteht.

12. Versuchskit zum Nachweis von Antibiotika in einer biologischen Flüssigkeit durch das Verfahren gemäß einem der Ansprüche 1 bis 11, umfassend wenigstens ein Erkennungsmittel, das gegen die Antibiotika mit β-Lactamkern empfindlich ist, erhalten aus Bacillus licheniformis, und wenigstens ein auf einem Träger immobilisiertes Referenzantibiotikum, **dadurch gekennzeichnet, dass** das gegen die Antibiotika mit β-Lactamkern empfindliche Erkennungsmittel der Rezeptor BlaR oder der Rezeptor BlaR-CTD, erhalten aus Bacillus licheniformis, ist.
